# EUROPÄISCHE PATENTANMELDUNG

(11) **EP 4 678 196 A1**
(43) Veröffentlichungstag der Anmeldung: **14.01.2026**
(21) Anmeldenummer: 24187301.7
(22) Anmeldetag: 09.07.2024
(51) Int. Cl.: A61L 24/00, A61L 24/04

(54) **PASTENFÖRMIGES HÄMOSTYPTIKUM, SEINE VERWENDUNG UND EIN VERFAHREN ZUR HERSTELLUNG EINES LOKALEN WIRKSTOFFFREISETZUNGSSYSTEMS**

(71) Anmelder: Heraeus Medical GmbH, 61273 Wehrheim (DE)
(72) Erfinder: Vogt, Sebastian, 61273 Wehrheim (DE); kluge, Thomas, 61273 Wehrheim (DE)
(74) Vertreter: Heraeus IP

(57) **Zusammenfassung**

Es wird ein pastenförmiges Hämostyptikum vorgeschlagen. Das Hämostyptikum umfasst
a) mindestens einen partikulären Zuckeralkohol;
b) mindestens einen gesättigten Glycerintrifettsäureester mit einem Schmelzpunkt größer/gleich 40°C, und
c) mindestens einen gesättigten Glycerintrifettsäureester mit einem Schmelzpunkt von kleiner 0 °C und

Weiterhin werden die Verwendungen des pastenförmigen Hämostyptikums und ein Verfahren zu dessen Herstellung beschrieben.

## Beschreibung

### EINLEITUNG

Die vorliegende Erfindung betrifft ein pastenförmiges Hämostyptikum, das zur mechanischen Versiegelung von blutendem Knochengewebe einsetzbar ist. Weiterer Gegenstände der vorliegenden Erfindung ist ein Wirkstofffreisetzungssystem, welches auf dem pastenförmigen Hämostyptikum basiert. Weiterer Gegenstand sind die Verwendungen des erfindungsgemäßen Hämostyptikums und des erfindungsgemäßen Wirkstofffreisetzungssystems sowie Verfahren zur Herstellung des erfindungsgemäßen Hämostyptikums und des erfindungsgemäßen Wirkstofffreisetzungssystems.

### HINTERGRUND DER ERFINDUNG UND STAND DER TECHNIK

Bei Operationen wird die Blutstillung (Hämostase) je nach anatomischen Gegebenheiten durch unterschiedliche Verfahren, wie z. B. die Elektrokoagulation (Kauterisierung) der Blutgefäße, durchgeführt. Bei einer Reihe von Operationen im Schädelbereich und vor allem am Sternum wird bei den dort auftretenden starken Blutungen aufgrund der anatomischen Situation Knochenwachs zur Versiegelung der Kapillargefäße und damit der Blutstillung eingesetzt. Dazu wird das plastisch verformbare Knochenwachs vom Operateur direkt auf bzw. in die blutenden Knochenareale gedrückt. Dabei kommt es zu einer Stauung des Blutflusses, wodurch Hämatome entstehen und die versorgenden Gefäße durch Thrombozytenaggregation und durch Fibrin letztlich verschlossen werden.

Knochenwachs ist mindestens seit dem 19. Jahrhundert bekannt und enthält im Allgemeinen gebleichtes Bienenwachs und einen Weichmacher. Als Weichmacher werden unter anderem Mandelöl, Vaseline, Palmitinsäure, Isopropylester und Myristinsäureisopropylester verwendet. Knochenwachse auf der Basis von Bienenwachs gelten als nicht biodegradierbar im humanen Organismus. Auf Grund der chemischen Zusammensetzung des verwendeten Bienenwachses wird Knochenwachs durch humane Enzyme nicht abgebaut. Dadurch verbleibt das Knochenwachs nach der Blutstillung im beziehungsweise am Knochengewebe und stellt eine Barriere für einwachsendes neues Knochengewebe dar.

Für die hämostyptische Wirkung der Knochenwachse ist deren gute Haftung auf feuchtem und auch fettigem Knochengewebe und deren hohen Zähigkeit ursächlich. Die gegenwärtig im Handel erhältlichen Knochenwachse weisen eine sehr gute hämostyptische Wirkung auf.

Allerdings gibt es häufig über längere Zeiträume unerwünschte Neben- und Folgewirkungen im humanen Organismus (S. E. Katz, J. Rotmann: Adverse effects of bone wax in surgery of the orbit. Ophthal Plast. Reconstr. 1996, 12 (2) 121-126.; M. Lavigne et al.: Bone-wax granuloma alter femoral neckosteoplasty. Can. J. Surg. 2008, 51 (3) E58-60.; R. T. Allison: Foreign body reactions and an associated histological artifact due to bone wax. Br. J. Biomed. Sci. 1994, 51 (1) 14-17.; O. Eser et al.: Bone wax as a cause of foreign body reaction alter lumbar disc surgery: A case report. Adv. Ther. 2007, 24 (3) 594-7.).

Es sind Alternativen zu herkömmlich zusammengesetzten Knochenwachs bekannt.

EP 0 109 310 A offenbart eine wachsartige Masse, die auf Calciumfettsäuresalzen und Oligomeren von Hydroxycarbonsäuren basiert.

Aus den Schriften US 4,595,713 A, DE 322 95 40 A, DE 382 52 11 A und EP 1 142 597 A sind wachsartige Zusammensetzungen bekannt, die Oligoester von Hydroxycarbonsäuren, wie zum Beispiel Milchsäure und 6-Hydroxycarbonsäure, enthalten. Es hat sich gezeigt, dass beim Einsatz dieser wachsartigen Zusammensetzungen während des hydrolytischen Abbaus saure Degradationsprodukte entstehen, die das Knochengewebe infolge einer lokalen pH-Wert Absenkung beeinträchtigen können.

Eine Alternative stellen Zusammensetzungen auf der Basis von Polyethern dar (US 2009/286886 A und US 2011/002974 A). Als Polyether können beispielsweise Poly(propylenglykol-co-ethylenglykole) verwendet werden. Diese Zusammensetzungen sind bei Handwärme knetbar und streichfähig. Als nachteilig ist jedoch die gute Löslichkeit dieser Polyether in wässrigem Milieu zu sehen. Dies führt dazu, dass die Haftung dieser Zusammensetzungen im Fall von stark blutendem Knochengewebe infolge der Anlösung der wachsartigen Zusammensetzung erschwert sein kann. Dadurch kann es unter Umständen zu Nachblutungen kommen, die wiederum eine schnelle Anlösung oder Auflösung der Siegelung bedingen. Vorteilhaft an diesen Gemischen ist jedoch, dass sie keinerlei Barrierefunktion für die Knochenheilung besitzen und vollständig renal ausgeschieden werden (A. Suwan et al.: Controversial role of two different local haemostatic agents on bone healing. J. am Sci. 2010, 6 (12) 155-163.).

In den Patentschriften DE 10 2011 016277 B und DE 10 2011 122 752 B wird ein pastenförmiges Hämostyptikum beschrieben. Das Hämostyptikum ist aufgebaut aus (a) wenigstens einen gesättigten Glycerin-1,2,3-trifettsäureester mit einer Schmelztemperatur von mehr als 37 °C, (b) wenigstens einen zumindest teilweise in partikulärer Form vorliegenden Füllstoff mit einer Schmelztemperatur von mehr als 37 °C und (c) wenigstens einer Verbindung mit einer Schmelztemperatur von nicht mehr als 37 °C, die bei einer Temperatur von 25 °C eine Löslichkeit von weniger als 50 Gramm pro Liter Wasser aufweist. Als Füllstoffe werden Polymere von wenigstens einem Alkylenoxid, Copolymeren von wenigstens einem Alkylenoxid und Calciumverbindungen vorgeschlagen. Als Calciumverbindungen werden Calciumcarbonat, Dolomit, α-Tricalciumcarbonat, β-Tricalciumcarbonat, Hydroxylapatit, Carbonatapatit, Octacalciumphosphat, amorphisiertem Calciumphosphat, Calciumsulfatdihydrat und Calciumsulfathemihydrat beschrieben. Als Polymere sind Polyethylenglykole und Poly(propylenglykol-co-ethylenglycol) (Poloxamer) bevorzugt. Flüssige Fettsäureester stellten die dritte Komponente dar. Es zeigte sich in eigenen Untersuchungen zur *in* vitro-Cytotoxizität gemäß der aktuellen ISO1993-5, dass ein Hämostyptikum gemäß den Patentschriften DE 10 2011 016 277 B und DE 10 2011 122 752 B, das aus einem festen Glycerintrifettsäureester, einem flüssigen Glycerintrifettsäureester und den Füllstoffen Calciumcarbonat und Calciumsulfat zusammengesetzt ist, nicht cytotoxisch gemäß ISO10993-5 ist. Dagegen sind Hämostyptika in ähnlicher Zusammensetzung, jedoch mit Poly(propylenglykol-co-ethylenglycol) (Poloxamer) als Füllstoff anstelle der anorganischen Calciumsalze, eindeutig cytotoxisch. Die Calciumverbindungen sind darüber hinaus aufgrund ihrer abrasiven Eigenschaften nachteilig.

Es besteht daher Bedarf an einem plastisch verformbaren, biodegradierbaren Hämostyptikum, das die vorstehend beschriebenen Nachteile im Allgemeinen nicht aufweist.

Insbesondere besteht ein Bedarf an pastenförmigen Hämostyptika, die vollständig aus nicht abrasiven organischen, biokompatiblen Substanzen bestehen. Abrasive Bestandteile sind insbesondere kritisch zu sehen, wenn das Hämostyptikum in der Nähe von Gelenkendoprothesen verwendet wird, weil diese die Gleitflächen abrasiv schädigen können, wenn die abrasiven Partikel zwischen die artikulierenden Flächen der Gelenkendoprothesen kommen.

### AUFGABE DER ERFINDUNG

Aufgabe der Erfindung ist die Entwicklung eines pastenförmigen Hämostyptikums, dass vorzugsweise aus biokompatiblen und resorbierbaren Materialien besteht und keine *in* vitro-Cytotoxizität gemäß der ISO10993-5 besitzt. In diesem Zusammenhang ist es auch besonders bevorzugt, wenn das erfindungsgemäße pastenförmige Hämostyptikum keine amphiphilen Eigenschaften aufweist. Amphiphile Stoffe durchdringen sowohl hydro- als auch lipophile Bestandteile einer Zelle und schwächen so die Zellmembran, können also die Cytotoxizität steigern. Es wird weiterhin angestrebt, dass es keine abrasiven, anorganischen Calciumsalze enthält. Das zu entwickelnde Hämostyptikum sollte zusätzlich keine Komponenten enthalten, die von Mikroorganismen als Energiequelle genutzt werden können. Weiterhin sollte das Hämostyptikum keine aciden oder basischen Bestandteile in größeren Mengen abgeben, um das Knochengewebe nicht durch einen nicht physiologischen pH-Wert zu schädigen. Darüber hinaus sollte das Material biodegradierbar sein oder renal ausgeschieden werden können, damit keine dauerhafte Barrierewirkung durch das Material den Heilungsprozess des Knochengewebes behindern kann. Das Hämostyptikum soll leicht manuell plastisch verformt werden können. Außerdem sollte das pastenförmige Material beim Kneten und beim Applizieren nicht an Gummihandschuhen haften bleiben. Es soll auf feuchtem Knochengewebe und auch auf den Oberflächen von metallischen Implantaten anhaften. Außerdem wird angestrebt, dass das Hämostyptikum mit beliebigen pulverförmigen pharmazeutischen Wirkstoffen vermischt werden kann, ohne dass die Haftungseigenschaften und die plastische Verformbarkeit des Hämostyptikums wesentlich beeinträchtigt werden. Die Zähigkeit dieses Hämostyptikums sollte so hoch sein, dass das pastenförmige Material dem Blutungsdruck standhält. Ferner sollte das Hämostyptikum eine hinreichend große Kohäsion aufweisen, so dass es bei Kontakt mit Blut oder anderen wässrigen Flüssigkeiten nicht auseinanderfällt oder sich innerhalb weniger Minuten auflöst.

Die Aufgaben der Erfindung werden zunächst durch das erfindungsgemäße Hämostyptikum gelöst.

### ZUSAMMENFASSUNG DER ERFINDUNG

Gegenstand der vorliegenden Erfindung ist zunächst ein pastenförmiges Hämostyptikum. Das Hämostyptikum ist dadurch gekennzeichnet, dass es
a) mindestens einen partikulären Zuckeralkohol,
b) mindestens einen gesättigten Glycerintrifettsäureesters mit einem Schmelzpunkt größer/gleich 40 °C,
c) mindestens einen gesättigten Glycerintrifettsäureesters mit einem Schmelzpunkt von kleiner 0 °C
umfasst.

Die einzelnen Bestandteile des erfindungsgemäßen Hämostyptikums werden weiter unten beschrieben.

In einer bevorzugten Ausgestaltung umfasst das erfindungsgemäße Hämostyptikum, jeweils bezogen auf das Gesamtgewicht des Hämostyptikums,
a) 30-60 Gew.-%, bevorzugt 40-60 Gew.-%, noch bevorzugter 50-60 Gew.-%, mindestens eines partikulären Zuckeralkohols,
b) 20-35 Gew.-%, bevorzugt 20-30 Gew.-%, noch bevorzugter 20-28 Gew.-%, mindestens eines gesättigten Glycerintrifettsäureesters mit einem Schmelzpunkt grö-βer/gleich 40 °C, und
c) 20-35 Gew.-%, bevorzugt 20-30 Gew.-%, noch bevorzugter 20-28 Gew.-%., mindestens eines gesättigten Glycerintrifettsäureesters mit einem Schmelzpunkt von kleiner 0 °C.

Das erfindungsgemäße Hämostyptikum weist beispielsweise die folgenden Vorteile auf.

Das erfindungsgemäße pastenförmige Hämostyptikum ist vorzugsweise nicht zytotoxisch gemäß der Norm ISO10993-5, bestimmt durch den MTT-Test ((3-(4,5-Dimethylthiazol-2-yl)-2,5-diphenyltetrazoliumbromid)-Test) oder durch den XTT-Test (((Natrium-3'-[1-(phe-nylamincarbonyl)-3,4-tetrazolium]-bis-(4-methoxy-6-nitro))-benzolsulfonsäurehydrat)-Test).

Das erfindungsgemäße pastenförmige Hämostyptikum zeichnet sich durch seine plastische Verformbarkeit, Biodegradierbarkeit und Beständigkeit gegenüber dem flüssigen, insbesondere wässrigen Milieu aus. Es bleibt in Abwesenheit äußerer Krafteinwirkung form- und volumenstabil, insbesondere *ex vivo.* Hiervon unberührt ist das Hämostyptikums degradierbar, wie hierin beschrieben.

Das erfindungsgemäße pastenförmige Hämostyptikum ist zur Behandlung von beschädigtem Knochengewebe geeignet und kann sowohl zur mechanischen Hämostase als auch als lokales pastenförmiges Wirkstofffreisetzungszentrum dienen. Das erfindungsgemäße Hämostyptikum haftet im Allgemeinen an Knochen, Glas und Metall gleichermaßen.

Zusätzlich ist es möglich, dem erfindungsgemäßen Hämostyptikum einen festen oder vorzugsweise partikulären pharmazeutischen Wirkstoff hinzuzusetzen. In dieser Ausgestaltung kann das Hämostyptikum, wenn es zur Behandlung von Schäden am Knochengewebe verwendet wird, als lokales pastenförmiges Wirkstofffreisetzungszentrum dienen.

Das erfindungsgemäße pastenförmige Hämostyptikum ist im Allgemeinen durch einfaches Verkneten seiner Bestandteile im Labor- oder Industriemaßstab zugänglich.

### DETAILLIERTE BESCHREIBUNG

### i) Pastenförmiges Hämostyptikum

Erfindungsgemäß wird ein Hämostyptikum zur Verfügung gestellt.

Unter einem Hämostyptikum werden erfindungsgemäß Zusammensetzungen verstanden, die blutstillende Eigenschaften aufweisen.

Die Erfindung basiert auf der überraschenden Erkenntnis, dass Gemische der wie vorstehend definierten Komponenten a), b) und c) ein pastenförmiges Hämostyptikum bilden, das zur Versiegelung von blutendem Knochengewebe eingesetzt werden kann. Besonders überraschend ist dabei, dass das erfindungsgemäße Hämostyptikum als wachsartige, knetbare Masse vorliegt, die im Allgemeinen sowohl auf trockenen als auch auf feuchten Oberflächen haftet. Das Hämostyptikum haftet insbesondere auf metallischen Oberflächen, auf Glas und auf Knochengewebe. Die Zähigkeit und die mechanische Stabilität dieser Mischung sind überraschenderweise so hoch, dass diese als effektives Hämostyptikum für die Blutstillung verwendet werden kann und den bei Verletzungen vorherrschenden Blutungsdrücken standhält. Obgleich die Mischung im Allgemeinen biodegradierbar ist, weist sie überraschenderweise eine derart große mechanische Stabilität auf, dass sie beim Kontakt mit Wasser oder wässrigen Lösungen wie Blut nicht zerfällt.

Als biodegradierbar werden im Rahmen der vorliegenden Erfindung vorliegend Stoffe bezeichnet, die vom humanen Organismus abgebaut und/oder renal ausgeschieden werden können.

Das erfindungsgemäße Hämostyptikum umfasst mindestens einen Zuckeralkohol.

Zuckeralkohole, wie zum Bespiel Mannitol, wirken sich im Allgemeinen nicht auf den Insulinspiegel von Patienten aus, die mit dem erfindungsgemäßen Hämostyptikum behandelt werden. Die Zuckeralkohole des erfindungsgemäßen Hämostyptikums haben außerdem einen überraschenden Einfluss auf dessen pastenförmige Beschaffenheit. Ohne an eine Theorie gebunden zu sein, vermindern sie das Kristallwachstum der anderen möglichen Bestandteile während des Herstellungsvorgangs des erfindungsgemäßen pastenförmigen Hämostyptikums, indem sie gebildete Kristallitflächen benetzen und so für weitere kristallbildende Moleküle unzugänglich machen. Dadurch bleiben die Kristallite klein und das pastenförmige Hämostyptikum bildet keine Brocken, sondern verbleibt in der erfindungsgemäßen Pastenform, die eine einheitliche Konsistenz aufweist. Erfindungsgemäß wird davon ausgegangen, dass Zuckeralkohole vom menschlichen Körper unzersetzt renal mit dem Blut ausgeschieden werden, ohne einen Schaden zu verursachen. Zusätzlich haben sie insbesondere einen positiven Einfluss auf die Beschaffenheit des pastenförmigen Hämostyptikums und sind damit ein überraschend nützlicher Bestandteil des erfindungsgemäßen pastenförmigen Hämostyptikums.

Das erfindungsgemäße Hämostyptikum umfasst als weiteren Bestandteil Glycerintrifettsäureester. Im Rahmen der vorliegenden Erfindung werden unter Glycerintrifettsäureestern organische Verbindungen verstanden, die durch Lipasen zu Glycerin und Fettsäuren gespalten werden. Glycerin wird als natürlicher Bestandteil des humanen Organismus im Allgemeinen über Pyruvat und über den Citronensäurezyklus zu Kohlendioxid und Wasser abgebaut. Fettsäuren sind ebenfalls natürliche Bestandteile des humanen Organismus und werden im Fall von Fettsäuren mit einer geradzahligen Anzahl von C-Atomen über β-Oxidation im Allgemeinen vollständig zu Kohlendioxid und Wasser abgebaut.

Nähere Definitionen zu den einzelnen Bestandteilen des erfindungsgemäßen Hämostyptikums sind unten zu finden.

Das erfindungsgemäße Hämostyptikum ist plastisch verformbar. Unter plastischer Verformbarkeit wird vorliegend die Fähigkeit des Hämostyptikums verstanden, sich unter einer Krafteinwirkung irreversibel zu verformen und diese Form nach der Krafteinwirkung beizubehalten.

Ein weiterer Vorteil des erfindungsgemäßen pastenförmigen Hämostyptikums ist, dass es im Allgemeinen keine zytotoxischen Eigenschaften aufweist. Im Rahmen der vorliegenden Erfindung wird dieses vorzugsweise dadurch verwirklicht, dass lediglich solche Stoffe bzw. Stoffgemische in dem erfindungsgemäßen Hämostyptikum verwendet werden, die eine *in* vitro-Cytotoxizität gemäß ISO10993-5 mit einer Viability von größer 70 % bei 100% v/v beim MTT-Test ((3-(4,5-Dimethylthiazol-2-yl)-2,5-diphenyltetrazoliumbromid)-Test) oder beim XTT-Test (((Natrium-3'-[1-(phenylamincarbonyl)-3,4-tetrazolium]-bis-(4-methoxy-6-nitro))-benzolsulfon-säurehydrat)-Test) aufweisen. Das bedeutet, dass beim MTT- oder XTT-Test nach der ISO10993-5 das erfindungsgemäße pastenförmige Hämostyptikum eine Viability von vorzugsweise größer als 70,0 %, 70,5 %, 71,0 %, 71,5 %, 72,0 %, 72,5 %, 73,0 %, 73,5 %, 74,0 %, 74,5 %, 75,0 %, 75,5 %, 76,0 %, 76,5 %, 77,0 %, 77,5 %, 78,0 %, 78,5 %, 79,0 %, 79,5 %, 80,0 %, 80,5 %, 81,0 %, 81,5 %, 82,0 %, 82,5 %, 83,0 %, 83,5 %, 84,0 %, 84,5 %, 85,0 %, 85,5 %, 86,0 %, 86,5 %, 87,0 %, 87,5 %, 88,0 %, 88,5 %, 89,0 %, 89,5 %, 90,0 %, 90,5 %, 91,0 %, 91,5 %, 92,0 %, 92,5 %, 93,0 %, 93,5 %, 94,0 %, 94,5 % oder 95,0 % bei 100 % v/v aufweist. Bevorzugt ist dabei, dass mindestens eine Viability zwischen 70,0 % bis 80,0 % bei 100 % v/v erreicht wird.

### ii) Zusammensetzung des pastenförmigen Hämostyptikums

Das pastenförmige Hämostyptikum umfasst mindestens einen partikulären Zuckeralkohol.

Unter einem Zuckeralkohol wird im Rahmen der vorliegenden Erfindung ein nichtcyclisches Polyol verstanden, welches an jedem ihrer Kohlenstoffatome eine Hydroxygruppe gebunden aufweist. Erfindungsgemäß geeignete Zuckeralkohole werden im Allgemeinen als Reduktionsprodukte von Kohlenhydraten (Zuckern), also durch Reduktion (Hydrierung) der Ketogruppe beziehungsweise der Aldehydgruppe von Zuckern, erhalten. Dadurch sind die Zuckeralkohole in der Regel deutlich oxidationsstabiler und lagerstabiler als die zu Grunde liegenden Zucker. Weiterhin beeinflussen diese im Allgemeinen nicht oder nur geringfügig die Blutzuckerkonzentration im humanen Organismus und sind von vielen Mikroorganismen nicht oder nur eingeschränkt als Energiequelle nutzbar.

Partikuläre Zuckeralkohole im Sinne der Erfindung sind bevorzugterweise Alditole und Ketole, wobei besonders D-Mannitol (CAS 69-65-8), D,L-Mannitol (CAS 87-78-5), D-Sorbitol (CAS 50-70-4), Isomalt (6-O-a-D-Glucopyranosyl-D-glucitol) (CAS 534-73-6), 1-O-a-D-Glucopyranosil-D-mannitol (CAS 20942-99-8), Erythrit (CAS 149-32-6) und Xylitol (CAS 87-99-0) bevorzugt sind. Weiter bevorzugt als Zuckeralkohole sind Erythrit, Xylitol, D-Mannitol und D,L-Mannitol. Ganz besonders bevorzugt werden im Rahmen der vorliegenden Erfindung D-Mannitol und D,L-Mannitol verwendet.

Erfindungsgemäße Versuche mit unterschiedlichen Zuckeralkoholen zeigen, dass D-Mannitol im Rahmen der vorliegenden Erfindung besonders geeignet ist hinsichtlich der haptischen Eigenschaften und hinsichtlich der *in* vitro-Cytotoxizität gemäß der ISO10993-5. D-Mannitol hat weiterhin keinen Einfluss auf den Blutzuckerspiegel und ist hämokompatibel.

Zuckeralkohole besitzen im Allgemeinen keine aciden oder basischen Gruppen. Daher beeinflussen sie den pH-Wert von wässrigen Lösungen oder Wasser praktisch nicht, wenn sie darin gelöst sind oder in Kontakt mit den wässrigen Lösungen oder mit Wasser kommen. Versuche zeigen, dass der pH-Wert von destilliertem Wasser, in dem die erfindungsgemäßen pastenförmigen Hämostyptika eingebracht waren, im Bereich von pH 6,0-6,8 liegt.

Im Sinne der Erfindung liegen die Zuckeralkohole als Bestandteil a) vorzugsweise in Form von partikulären Zuckeralkoholen vor. Das bedeutet, dass die partikulären Zuckeralkohole vorzugsweise in Form fester Partikel vorliegen, die dadurch gekennzeichnet sind, dass sie über einen Partikeldurchmesser von vorzugsweise kleiner als 100 µm verfügen. Die Partikel des mindestens einen partikulären Zuckeralkohols sind also kleiner als vorzugsweise 100 µm, 99 µm, 98 µm, 97 µm, 96 µm, 95 µm, 94 µm, 93 µm, 92 µm, 91 µm, 90 µm, 89 µm, 88 µm, 87 µm, 86 µm, 85 µm, 84 µm, 83 µm, 82 µm, 81 µm, 80 µm, 79 µm, 78 µm, 77 µm, 76 µm, 75 µm, 74 µm, 73 µm, 72 µm, 71 µm, 70 µm, 69 µm, 68 µm, 67 µm, 66 µm, 65 µm, 64 µm, also 63 µm, 62 µm, 61 µm, 60 µm, 59 µm, 58 µm, 57 µm, 56 µm, 55 µm, 54 µm, 53 µm, 52 µm, 51 µm oder 50 µm. Der Partikeldurchmesser wird im Rahmen der vorliegenden Erfindung durch Siebfraktionierung bestimmt.

Je kleiner die Partikelgröße des Zuckeralkohols ist, umso geschmeidiger ist die daraus herstellbare Paste.

Ein weiterer Bestandteil des pastenförmigen Hämostyptikums im Sinne der vorliegenden Erfindung sind gesättigte Glycerintrifettsäureester mit einem Schmelzpunkt grö-βer/gleich 40 °C als Bestandteil b). Solche gesättigten Glycerintrifettsäureester sind im Allgemeinen bei Raumtemperatur fest. Des Weiteren liegt ihr Schmelzpunkt über der gewöhnlichen Körpertemperatur von Menschen von etwa 36 bis 38 °C, wodurch sie einen möglichen Bestandteil des erfindungsgemäßen Hämostyptikums darstellen, der auch unter in-vivo Bedingungen fest ist.

Als gesättigte Glycerintrifettsäureester mit einem Schmelzpunkt größer/gleich 40 °C im Sinne der vorliegenden Erfindung eignen sich insbesondere Glycerintristearat (CAS 55-43-1), Glycerintripalmitat (CAS 555-44-2), Glycerintrilaurat (CAS 555-44-2) und gemischte Glycerintrifettsäureester, die aus Stearat und/oder Palmitat und/oder Laurat und Glycerin gebildet sind. Denkbar sind aber auch andere mögliche Arten von gesättigten Fettsäureestern, die einen Schmelzpunkt größer/gleich 40 °C aufweisen und die zuvor beschriebenen, bevorzugten Eigenschaften des erfindungsgemäßen Hämostyptikums hinsichtlich Oberflächenhaftung, Verformbarkeit, Zähigkeit, Biodegradierbarkeit etc. ermöglichen. Diese gesättigten Fettsäureester mit einem Schmelzpunkt grö-βer/gleich 40 °C sind für die Anhaftung des Hämostyptikums an Knochengewebe und an metallischen Oberflächen bevorzugt. Weiterhin haben diese gesättigten Fettsäureester im Allgemeinen eine hohe Lagerstabilität und eine hohe Resistenz gegen Gammabestrahlung, weil sie keine Doppelbindungen oder andere oxidationsempfindliche Strukturen aufweisen. Daher ist das erfindungsgemäße partikuläre Hämostyptikum besonders geeignet, um Verletzungen oder operativ bedingte Beeinträchtigungen der Knochen zu behandeln. Etwaige auf die Behandlung mit dem erfindungsgemäßen partikulären Hämostyptikum folgende Röntgenaufnahmen schädigen die mit dem partikulären Hämostyptikum behandelte Stelle im Allgemeinen nicht. Insbesondere können mit dem erfindungsgemäßen Hämostyptikum auch Schäden an Knochen solcher Patienten behandelt werden, die danach einer Strahlentherapie ausgesetzt werden müssen. Die Bestrahlung kann das erfindungsgemäße partikuläre Hämostyptikum vorzugsweise nicht beeinträchtigen.

Eine weitere Komponente des pastenförmigen Hämostyptikums im Sinne der vorliegenden Erfindung sind die gesättigten Glycerintrifettsäureester mit einem Schmelzpunkt unter 0 °C als Bestandteil c). Solche gesättigten Glycerintrifettsäureester sind bei Raumtemperatur, also zwischen 20 °C und 30 °C flüssig.

Als gesättigte Glycerintrifettsäureester mit einem Schmelzpunkt unter 0 °C im Sinne der vorliegenden Erfindung eignen sich insbesondere Glycerintrioctanoat (CAS 538-23-8) und Glycerintridecanoat (CAS 612-71-6), wobei Gemische von Glycerinfettsäuren besonders bevorzugt und die Glycerinfettsäuregemische Miglyol 812 N (CAS 73398-61-5), Miglyol 810 N (CAS 73398-61-5) und Miglyol 829 N (Cas 91744-56-8) ganz besonders bevorzugt sind. Diese flüssigen Fettsäureester enthalten ebenfalls keine Doppelbindungen oder sonstigen oxidationsempfindlichen Strukturen und sind daher im Allgemeinen lagerstabil und weitgehend resistent gegen Gammastrahlung. Besonders vorteilhaft ist die Verwendung von Glycerintrifettsäureestern, deren Fettsäurereste eine gerade Anzahl von C-Atomen besitzen. Bei einer geraden Anzahl von C-Atomen werden Fettsäuren im humanen Organismus vollständig zu Kohlendioxid und Wasser verstoffwechselt.

Erfindungsgemäß ist es bevorzugt, wenn die unterschiedlichen Komponenten a) (partikuläre Zuckeralkohole) b) (gesättigte Glycerinfettsäureester mit einem Schmelzpunkt größer/gleich 40 °C) und c) (gesättigte Glycerinfettsäureester mit einem Schmelzpunkt unter 0 °C) jeweils zu einem bestimmten Anteil in dem erfindungsgemäßen Hämostyptikum vorliegen. Es ist erfindungsgemäß möglich, die jeweiligen Anteile der Komponenten am pastenförmigen Hämostyptikum in Abhängigkeit der ausgewählten Substanzen sowie der angedachten Anwendung des pastenförmigen Hämostyptikums zu variieren.

Im Sinne der vorliegenden Erfindung hat sich gezeigt, dass die Komponente a) bevorzugterweise 30 Gew.-% bis 60 Gew.-% an der Gesamtmasse des pastenförmigen Hämostyptikums ausmacht. Das bedeutet, dass der partikuläre Zuckeralkohol unabhängig von den anderen Bestandteilen einen Anteil von vorzugsweise mindestens 30 Gew.-%, im Allgemeinen mindestens 31 Gew.-%, im Allgemeinen mindestens 32 Gew.-%, im Allgemeinen mindestens 33 Gew.-%, im Allgemeinen mindestens 34 Gew.-%, im Allgemeinen mindestens 35 Gew.-%, im Allgemeinen mindestens 36 Gew.-%, im Allgemeinen mindestens 37 Gew.-%, im Allgemeinen mindestens 38 Gew.-%, im Allgemeinen mindestens 39 Gew.-%, im Allgemeinen mindestens 40 Gew.-%, im Allgemeinen mindestens 41 Gew.-%, im Allgemeinen mindestens 42 Gew.-%, im Allgemeinen mindestens 43 Gew.-%, im Allgemeinen mindestens 44 Gew.-%, im Allgemeinen mindestens 45 Gew.-%, im Allgemeinen mindestens 46 Gew.-%, im Allgemeinen mindestens 47 Gew.-%, im Allgemeinen mindestens 48 Gew.-%, im Allgemeinen mindestens 49 Gew.-% im Allgemeinen mindestens 50 Gew.-%, jeweils bezogen auf die Gesamtmasse des erfindungsgemäßen Hämostyptikums, ausmacht. Ferner bedeutet dieses, dass der partikuläre Zuckeralkohol unabhängig von den anderen Bestandteilen einen Anteil von vorzugsweise höchstens 60 Gew.-%, im Allgemeinen höchstens 59 Gew.-%, im Allgemeinen höchstens 58 Gew.-%, im Allgemeinen höchstens 57 Gew.-%, im Allgemeinen höchstens 56 Gew.-%, im Allgemeinen höchstens 55 Gew.-%, im Allgemeinen höchstens 54 Gew.-%, im Allgemeinen höchstens 53 Gew.-%, im Allgemeinen höchstens 52 Gew.-%, im Allgemeinen höchstens 51 Gew.-%, im Allgemeinen höchstens 50 Gew.-%, jeweils bezogen auf die Gesamtmasse des erfindungsgemäßen Hämostyptikums, ausmacht. Besonders bevorzugt ist es, wenn die Komponente a), also der partikuläre Zuckeralkohol, in einem Bereich von 40 Gew.-% bis 60 Gew.-%, noch bevorzugter von 50 Gew.-% bis 60 Gew.-%, jeweils bezogen auf die Gesamtmasse des erfindungsgemäßen Hämostyptikums, vorhanden ist.

Im Sinne der vorliegenden Erfindung hat sich gezeigt, dass die Komponenten b) und c) bevorzugterweise jeweils 20 Gew.-% bis 35 Gew.-% an der Gesamtmasse des pastenförmigen Hämostyptikums ausmachen.

Das bedeutet, dass der gesättigte Glycerintrifettsäureester mit einem Schmelzpunkt größer/gleich 40 °C unabhängig von den anderen Bestandteilen einen Anteil von vorzugsweise mindestens 20 Gew.-%, im Allgemeinen mindestens 21 Gew.-%, im Allgemeinen mindestens 22 Gew.-%, im Allgemeinen mindestens 23 Gew.-%, im Allgemeinen mindestens 24 Gew.-%, im Allgemeinen mindestens 25 Gew.-%, im Allgemeinen mindestens 26 Gew.-%, im Allgemeinen mindestens 27 Gew.-%, im Allgemeinen mindestens 28 Gew.-%, im Allgemeinen mindestens 29 Gew.-%, im Allgemeinen mindestens 30 Gew.-%, jeweils bezogen auf die Gesamtmasse des erfindungsgemäßen Hämostyptikums, ausmacht. Ferner bedeutet dieses, dass der gesättigte Glycerintrifettsäureester mit einem Schmelzpunkt größer/gleich 40 °C unabhängig von den anderen Bestandteilen einen Anteil von vorzugsweise höchstens 35 Gew.-%, im Allgemeinen höchstens 34 Gew.-%, im Allgemeinen höchstens 33 Gew.-%, im Allgemeinen höchstens 32 Gew.-%, im Allgemeinen höchstens 31 Gew.-%, im Allgemeinen höchstens 30 Gew.-%, jeweils bezogen auf die Gesamtmasse des erfindungsgemäßen Hämostyptikums, ausmacht.

Das bedeutet außerdem, dass der gesättigte Glycerintrifettsäureester mit einem Schmelzpunkt kleiner 0 °C unabhängig von den anderen Bestandteilen einen Anteil von vorzugsweise mindestens 20 Gew.-%, im Allgemeinen mindestens 21 Gew.-%, im Allgemeinen mindestens 22 Gew.-%, im Allgemeinen mindestens 23 Gew.-%, im Allgemeinen mindestens 24 Gew.-%, im Allgemeinen mindestens 25 Gew.-%, im Allgemeinen mindestens 26 Gew.-%, im Allgemeinen mindestens 27 Gew.-%, im Allgemeinen mindestens 28 Gew.-%, im Allgemeinen mindestens 29 Gew.-%, im Allgemeinen mindestens 30 Gew.-%, jeweils bezogen auf die Gesamtmasse des erfindungsgemä-ßen Hämostyptikums, ausmacht. Ferner bedeutet dieses, dass der gesättigte Glycerintrifettsäureester mit einem Schmelzpunkt kleiner 0 °C unabhängig von den anderen Bestandteilen einen Anteil von vorzugsweise höchstens 35 Gew.-%, im Allgemeinen höchstens 34 Gew.-%, im Allgemeinen höchstens 33 Gew.-%, im Allgemeinen höchstens 32 Gew.%, im Allgemeinen höchstens 31 Gew.-%, im Allgemeinen höchstens 30 Gew.-%, jeweils bezogen auf die Gesamtmasse des erfindungsgemäßen Hämostyptikums, ausmacht.

Das erfindungsgemäße pastenförmige Hämostyptikum umfasst insbesondere, jeweils bezogen auf das Gesamtgewicht des pastenförmige Hämostyptikums,
a) 30-60 Gew.-%, bevorzugt 40-60 Gew.-%, noch bevorzugter 50-60 Gew.-%, mindestens eines partikulären Zuckeralkohols, ausgewählt aus der Gruppe, bestehend aus D-Mannitol, D,L-Mannitol, Xylitol und Erythrit;
b) 20-35 Gew.-%, bevorzugt 20-30 Gew.-%, noch bevorzugter 20-28 Gew.-%, mindestens eines gesättigten Glycerintrifettsäureesters mit einem Schmelzpunkt grö-βer/gleich 40 °C, ausgewählt aus der Gruppe, bestehend aus Glycerintripalmitat, Glycerintristearat und Glycerintribehenat,
c) 20-35 Gew.-%, bevorzugt 20-30 Gew.-%, noch bevorzugter 20-28 Gew.-%, mindestens eines gesättigten Glycerintrifettsäureesters mit einem Schmelzpunkt von kleiner 0 °C, ausgewählt aus der Gruppe, bestehend aus Glycerinfettsäuregemischen.

Das erfindungsgemäße pastenförmige Hämostyptikum umfasst insbesondere, jeweils bezogen auf das Gesamtgewicht des pastenförmige Hämostyptikums,
d) 30-60 Gew.-%, bevorzugt 40-60 Gew.-%, noch bevorzugter 50-60 Gew.-%, mindestens eines partikulären Zuckeralkohols, ausgewählt aus der Gruppe, bestehend aus D-Mannitol, D,L-Mannitol, Xylitol und Erythrit;
e) 20-35 Gew.-%, bevorzugt 20-30 Gew.-%, noch bevorzugter 20-28 Gew.-%, mindestens eines gesättigten Glycerintrifettsäureesters mit einem Schmelzpunkt grö-βer/gleich 40 °C, ausgewählt aus der Gruppe, bestehend aus Glycerintripalmitat, Glycerintristearat und Glycerintribehenat,
f) 20-35 Gew.-%, bevorzugt 20-30 Gew.-%, noch bevorzugter 20-28 Gew.-%, mindestens eines gesättigten Glycerintrifettsäureesters mit einem Schmelzpunkt von kleiner 0 °C, ausgewählt aus der Gruppe, bestehend aus den Glycerinfettsäuregemischen Miglyol 812 N, Miglyol 810 N und Miglyol 829 N.

Das erfindungsgemäße pastenförmige Hämostyptikum umfasst insbesondere, jeweils bezogen auf das Gesamtgewicht des pastenförmige Hämostyptikums,
a) 30-60 Gew.-%, bevorzugt 40-60 Gew.-%, noch bevorzugter 50-60 Gew.-%, mindestens eines partikulären Zuckeralkohols, ausgewählt aus der Gruppe, bestehend aus D-Mannitol und D,L-Mannitol;
b) 20-35 Gew.-%, bevorzugt 20-30 Gew.-%, noch bevorzugter 20-28 Gew.-%, mindestens eines gesättigten Glycerintrifettsäureesters mit einem Schmelzpunkt grö-βer/gleich 40 °C, ausgewählt aus der Gruppe, bestehend aus Glycerintripalmitat, Glycerintristearat und Glycerintribehenat; und
c) 20-35 Gew.-%, bevorzugt 20-30 Gew.-%, noch bevorzugter 20-28 Gew.-%, mindestens eines gesättigten Glycerintrifettsäureesters mit einem Schmelzpunkt von kleiner 0 °C, ausgewählt aus der Gruppe, bestehend aus Glycerinfettsäuregemischen.

Das erfindungsgemäße pastenförmige Hämostyptikum umfasst insbesondere, jeweils bezogen auf das Gesamtgewicht des pastenförmige Hämostyptikums,
a) 30-60 Gew.-%, bevorzugt 40-60 Gew.-%, noch bevorzugter 50-60 Gew.-%, mindestens eines partikulären Zuckeralkohols, ausgewählt aus der Gruppe, bestehend aus D-Mannitol und D,L-Mannitol;
b) 20-35 Gew.-%, bevorzugt 20-30 Gew.-%, noch bevorzugter 20-28 Gew.-%, mindestens eines gesättigten Glycerintrifettsäureesters mit einem Schmelzpunkt grö-βer/gleich 40 °C, ausgewählt aus der Gruppe, bestehend aus Glycerintripalmitat, Glycerintristearat und Glycerintribehenat; und
c) 20-35 Gew.-%, bevorzugt 20-30 Gew.-%, noch bevorzugter 20-28 Gew.-%, mindestens eines gesättigten Glycerintrifettsäureesters mit einem Schmelzpunkt von kleiner 0 °C, ausgewählt aus der Gruppe, bestehend aus den Glycerinfettsäuregemischen Miglyol 812 N, Miglyol 810 N und Miglyol 829 N.

Das erfindungsgemäße pastenförmige Hämostyptikum wird insbesondere in vivo zur Behandlung aller denkbaren Arten von äußeren Schädigungen der Knochenstruktur verwendet.

Zu diesem Zweck kann es im Sinne der Erfindung besonders vorteilhaft sein, dem pastenförmigen Hämostyptikum neben den zuvor genannten Bestandteilen a), b) und c) mindestens einen festen, vorzugsweise einen partikulären, pharmazeutischen Wirkstoff hinzuzusetzen. Der feste bzw. partikuläre pharmazeutische Wirkstoff kann dabei dispergiert im pastenförmigen Hämostyptikum vorliegen, ohne seine chemische Gestalt geändert zu haben. Der Wirkstoff kann auf diese Weise vorzugsweise über einen längeren Zeitraum lokal abgegeben werden und dort alle möglichen denkbaren Heilwirkungen auf das etwaig geschädigte Knochengewebe in seiner Umgebung ausüben (*sustained release*). Wenn das erfindungsgemäßes Hämostyptikum einen Wirkstoff umfasst, resultiert ein erfindungsgemäße Wirkstofffreisetzungssystem. Der Wirkstoff kann entweder bei der Herstellung des Hämostyptikums hinzugefügt werden, oder kann von einem medizinischen Anwender unmittelbar vor der Verabreichung an einen Patienten hinzugefügt werden.

Daher betrifft die vorliegende Erfindung auch ein Wirkstofffreisetzungssystem, umfassend
a) mindestens einen partikulären Zuckeralkohol;
b) mindestens einen gesättigten Glycerintrifettsäureesters mit einem Schmelzpunkt größer/ gleich 40 °C;
c) mindestens einen gesättigten Glycerintrifettsäureesters mit einem Schmelzpunkt von kleiner 0 °C; und
d) einen partikulären pharmazeutischen Wirkstoff.

In einer bevorzugten Ausgestaltung umfasst das erfindungsgemäße Wirkstofffreisetzungssystem, jeweils bezogen auf das Gesamtgewicht des Wirkstofffreisetzungssystems,
a) 30-60 Gew.-%, bevorzugt 40-60 Gew.-%, noch bevorzugter 50-60 Gew.-%, mindestens eines partikulären Zuckeralkohols;
b) 20-35 Gew.-%, bevorzugt 20-30 Gew.-%, noch bevorzugter 20-28 Gew.-%, mindestens eines gesättigten Glycerintrifettsäureesters mit einem Schmelzpunkt grö-βer/gleich 40 °C;
c) 20-35 Gew.-%, bevorzugt 20-30 Gew.-%, noch bevorzugter 20-28 Gew.-%., mindestens eines gesättigten Glycerintrifettsäureesters mit einem Schmelzpunkt von kleiner 0 °C; und
d) 0,5-15 Gew.-%, bevorzugt 1-12 Gew.-%, noch bevorzugter 2-10 Gew.-%, eines partikulären pharmazeutischen Wirkstoffs.

Dazu ist prinzipiell jeder pharmazeutische Wirkstoff geeignet. Erfindungsgemäß sind vor Allem Antiinfektiva, Aktivatoren der Blutgerinnung, Fibrinolysehemmer, Immunmodulatoren, Steroidhormone und Wachstumsfaktoren bevorzugt. Ganz besonders Gentamicin (CAS 1403-66-3), Vancomycin (CAS 1404-90-6) , Tobramycin (CAS 32986-56-4), Clindamycin (Cas 18323-44-9), Colistin (CAS 1066-17-7), Meropenem (CAS 96036-03-29), Metronidazol (CAS 443-48-1), Caspofungin (CAS 162808-62-0), Fluconazol (Cas 86386-73-4), Amphotericin B (CAS 1397-89-3), Calciumgluconat (CAS 299-28-5), Tranexamsäure (CAS 11197-18-8), 6-Amino-capronsäure (CAS 60-32-2), p-Aminomethylbenzoesäure (CAS 150-13-0), Prednisolon (CAS 50-24-8), Dexamethason (CAS 50-02-2) und Cyclosporin A (CAS 79217-60-0) sind bevorzugt.

Daher betrifft die vorliegende Erfindung insbesondere ein Wirkstofffreisetzungssystem, umfassend, jeweils bezogen auf das Gesamtgewicht des Wirkstofffreisetzungssystems,
a) 30-60 Gew.-%, bevorzugt 40-60 Gew.-%, noch bevorzugter 50-60 Gew.-% mindestens eines partikulären Zuckeralkohols;
b) 20-35 Gew.-%, bevorzugt 20-30 Gew.-%, noch bevorzugter 20-28 Gew.-%, mindestens eines gesättigten Glycerintrifettsäureesters mit einem Schmelzpunkt grö-βer/gleich 40 °C;
c) 20-35 Gew.-%, bevorzugt 20-30 Gew.-%, noch bevorzugter 20-28 Gew.-% mindestens eines gesättigten Glycerintrifettsäureesters mit einem Schmelzpunkt von kleiner 0 °C; und
d) 0,5-15 Gew.-%, bevorzugt 1-12 Gew.-%, noch bevorzugter 2-10 Gew.-% eines partikulären pharmazeutischen Wirkstoffs, ausgewählt aus der Gruppe, bestehend aus Antiinfektiva, Aktivatoren der Blutgerinnung, Fibrinolysehemmer, Immunmodulatoren, Steroidhormone und Wachstumsfaktoren.

Ferner betrifft die vorliegende Erfindung ein Wirkstofffreisetzungssystem, umfassend, jeweils bezogen auf das Gesamtgewicht des Wirkstofffreisetzungssystems,
a) 30-60 Gew.-%, bevorzugt 40-60 Gew.-%, noch bevorzugter 50-60 Gew.-% mindestens eines partikulären Zuckeralkohols;
b) 20-35 Gew.-%, bevorzugt 20-30 Gew.-%, noch bevorzugter 20-28 Gew.-%, mindestens eines gesättigten Glycerintrifettsäureesters mit einem Schmelzpunkt grö-βer/gleich 40 °C;
c) 20-35 Gew.-%, bevorzugt 20-30 Gew.-%, noch bevorzugter 20-28 Gew.-% mindestens eines gesättigten Glycerintrifettsäureesters mit einem Schmelzpunkt von kleiner 0 °C; und
d) 0,5-15 Gew.-%, bevorzugt 1-12 Gew.-%, noch bevorzugter 2-10 Gew.-% eines partikulären pharmazeutischen Wirkstoffs, ausgewählt aus der Gruppe, bestehend aus Gentamicin, Vancomycin, Tobramycin, Clindamycin, Colistin, Meropenem, Metronidazol, Caspofungin, Fluconazol, Amphotericin B, Calciumgluconat, Tranexamsäure, 6-Aminocapronsäure, p-Aminomethylbenzoesäure, Prednisolon, Dexamethason, Daptomycin und Cyclosporin A.

Das erfindungsgemäße Wirkstofffreisetzungssystem enthält einen partikulären pharmazeutischen Wirkstoff, der aus den vorgenannten Wirkstoffen ausgewählt ist. Von diesen vorgenannten Wirkstoffen sind insbesondere die Antiinfektiva bevorzugt. Noch weiter bevorzugt sind Antibiotika, insbesondere die Antibiotika Gentamicin, Vancomycin, Clindamycin und Daptomycin.

Daher betrifft die vorliegende Erfindung insbesondere ein Wirkstofffreisetzungssystem, umfassend, jeweils bezogen auf das Gesamtgewicht des Wirkstofffreisetzungssystems,
a) 30-60 Gew.-%, bevorzugt 40-60 Gew.-%, noch bevorzugter 50-60 Gew.-% mindestens eines partikulären Zuckeralkohols;
b) 20-35 Gew.-%, bevorzugt 20-30 Gew.-%, noch bevorzugter 20-28 Gew.-% mindestens eines gesättigten Glycerintrifettsäureesters mit einem Schmelzpunkt grö-βer/gleich 40 °C;
c) 20-35 Gew.-%, bevorzugt 20-30 Gew.-%, noch bevorzugter 20-28 Gew.-% mindestens eines gesättigten Glycerintrifettsäureesters mit einem Schmelzpunkt von kleiner 0 °C; und
d) 0,5-15 Gew.-%, bevorzugt 1-12 Gew.-%, noch bevorzugter 2-10 Gew.-% ein partikuläres Antibiotikum.

Ferner betrifft die vorliegende Erfindung insbesondere ein Wirkstofffreisetzungssystem, umfassend, jeweils bezogen auf das Gesamtgewicht des Wirkstofffreisetzungssystems,
a) 30-60 Gew.-%, bevorzugt 40-60 Gew.-%, noch bevorzugter 50-60 Gew.-% mindestens eines partikulären Zuckeralkohols;
b) 20-35 Gew.-%, bevorzugt 20-30 Gew.-%, noch bevorzugter 20-28 Gew.-% mindestens eines gesättigten Glycerintrifettsäureesters mit einem Schmelzpunkt grö-βer/gleich 40 °C;
c) 20-35 Gew.-%, bevorzugt 20-30 Gew.-%, noch bevorzugter 20-28 Gew.-% mindestens eines gesättigten Glycerintrifettsäureesters mit einem Schmelzpunkt von kleiner 0 °C; und
d) 0,5-15 Gew.-%, bevorzugt 1-12 Gew.-%, noch bevorzugter 2-10 Gew.-% eines partikulären pharmazeutischen Wirkstoffs, ausgewählt aus der Gruppe, bestehend aus Gentamicin, Vancomycin, Clindamycin und Daptomycin.

Das erfindungsgemäße pastenförmige Wirkstofffreisetzungssystem umfasst insbesondere, jeweils bezogen auf das Gesamtgewicht des Wirkstofffreisetzungssystems,
a) 30-60 Gew.-%, bevorzugt 40-60 Gew.-%, noch bevorzugter 50-60 Gew.-%, mindestens eines partikulären Zuckeralkohols, ausgewählt aus der Gruppe, bestehend aus D-Mannitol, D,L-Mannitol, Xylitol und Erythrit;
b) 20-35 Gew.-%, bevorzugt 20-30 Gew.-%, noch bevorzugter 20-28 Gew.-%, mindestens eines gesättigten Glycerintrifettsäureesters mit einem Schmelzpunkt grö-βer/gleich 40 °C, ausgewählt aus der Gruppe, bestehend aus Glycerintripalmitat, Glycerintristearat und Glycerintribehenat;
c) 20-35 Gew.-%, bevorzugt 20-30 Gew.-%, noch bevorzugter 20-28 Gew.-%., mindestens eines gesättigten Glycerintrifettsäureesters mit einem Schmelzpunkt von kleiner 0 °C, ausgewählt aus der Gruppe, bestehend aus Glycerinfettsäuregemischen; und
d) 0,5-15 Gew.-%, bevorzugt 1-12 Gew.-%, noch bevorzugter 2-10 Gew.-% eines partikulären pharmazeutischen Wirkstoffs, ausgewählt aus der Gruppe, bestehend aus Gentamicin, Vancomycin, Clindamycin und Daptomycin.

Das erfindungsgemäße pastenförmige Wirkstofffreisetzungssystem umfasst insbesondere, jeweils bezogen auf das Gesamtgewicht des Wirkstofffreisetzungssystems,
a) 30-60 Gew.-%, bevorzugt 40-60 Gew.-%, noch bevorzugter 50-60 Gew.-%, mindestens eines partikulären Zuckeralkohols, ausgewählt aus der Gruppe, bestehend aus D-Mannitol, D,L-Mannitol, Xylitol und Erythrit;
b) 20-35 Gew.-%, bevorzugt 20-30 Gew.-%, noch bevorzugter 20-28 Gew.-%, mindestens eines gesättigten Glycerintrifettsäureesters mit einem Schmelzpunkt grö-βer/gleich 40 °C, ausgewählt aus der Gruppe, bestehend aus Glycerintripalmitat, Glycerintristearat und Glycerintribehenat,
c) 20-35 Gew.-%, bevorzugt 20-30 Gew.-%, noch bevorzugter 20-28 Gew.-%, mindestens eines gesättigten Glycerintrifettsäureesters mit einem Schmelzpunkt von kleiner 0 °C, ausgewählt aus der Gruppe, bestehend aus den Glycerinfettsäuregemischen Miglyol 812 N, Miglyol 810 N und Miglyol 829 N; und
d) 0,5-15 Gew.-%, bevorzugt 1-12 Gew.-%, noch bevorzugter 2-10 Gew.-%, jeweils bezogen auf die Gesamtmasse des erfindungsgemäßen Wirkstofffreisetzungssystem, eines partikulären pharmazeutischen Wirkstoffs, ausgewählt aus der Gruppe, bestehend aus Gentamicin, Vancomycin, Clindamycin und Daptomycin.

Das erfindungsgemäße pastenförmige Wirkstofffreisetzungssystem umfasst insbesondere, jeweils bezogen auf das Gesamtgewicht des Wirkstofffreisetzungssystems,
a) 30-60 Gew.-%, bevorzugt 40-60 Gew.-%, noch bevorzugter 50-60 Gew.-%, mindestens eines partikulären Zuckeralkohols, ausgewählt aus der Gruppe, bestehend aus D-Mannitol und D,L-Mannitol;
b) 20-35 Gew.-%, bevorzugt 20-30 Gew.-%, noch bevorzugter 20-28 Gew.-%, mindestens eines gesättigten Glycerintrifettsäureesters mit einem Schmelzpunkt grö-βer/gleich 40 °C, ausgewählt aus der Gruppe, bestehend aus Glycerintripalmitat, Glycerintristearat und Glycerintribehenat;
c) 20-35 Gew.-%, bevorzugt 20-30 Gew.-%, noch bevorzugter 20-28 Gew.-%., mindestens eines gesättigten Glycerintrifettsäureesters mit einem Schmelzpunkt von kleiner 0 °C, ausgewählt aus der Gruppe, bestehend aus Glycerinfettsäuregemischen; und
d) 0,5-15 Gew.-%, bevorzugt 1-12 Gew.-%, noch bevorzugter 2-10 Gew.-% eines partikulären pharmazeutischen Wirkstoffs, ausgewählt aus der Gruppe, bestehend aus Gentamicin, Vancomycin, Clindamycin und Daptomycin.

Das erfindungsgemäße pastenförmige Wirkstofffreisetzungssystem umfasst insbesondere, jeweils bezogen auf das Gesamtgewicht des Wirkstofffreisetzungssystems,
a) 30-60 Gew.-%, bevorzugt 40-60 Gew.-%, noch bevorzugter 50-60 Gew.-%, mindestens eines partikulären Zuckeralkohols, ausgewählt aus der Gruppe, bestehend aus D-Mannitol, und D,L-Mannitol;
b) 20-35 Gew.-%, bevorzugt 20-30 Gew.-%, noch bevorzugter 20-28 Gew.-%, mindestens eines gesättigten Glycerintrifettsäureesters mit einem Schmelzpunkt grö-βer/gleich 40 °C, ausgewählt aus der Gruppe, bestehend aus Glycerintripalmitat, Glycerintristearat und Glycerintribehenat,
c) 20-35 Gew.-%, bevorzugt 20-30 Gew.-%, noch bevorzugter 20-28 Gew.-%, mindestens eines gesättigten Glycerintrifettsäureesters mit einem Schmelzpunkt von kleiner 0 °C, ausgewählt aus der Gruppe, bestehend aus den Glycerinfettsäuregemischen Miglyol 812 N, Miglyol 810 N und Miglyol 829 N; und
d) 0,5-15 Gew.-%, bevorzugt 1-12 Gew.-%, noch bevorzugter 2-10 Gew.-%, jeweils bezogen auf die Gesamtmasse des erfindungsgemäßen Wirkstofffreisetzungssystem, eines partikulären pharmazeutischen Wirkstoffs, ausgewählt aus der Gruppe, bestehend aus Gentamicin, Vancomycin, Clindamycin und Daptomycin.

Erfindungsgemäß ist somit vorzugsweise vorgesehen, dass zur Ausbildung des erfindungsgemäßen Wirkstofffreisetzungssystem 0,5-15,0 Gew.-% mindestens eines festen pharmazeutischen Wirkstoffes, vorzugsweise eines partikulären pharmazeutischen Wirkstoffes, dem pastenförmigen Hämostyptikum zugesetzt werden. Das bedeutet, dass der pharmazeutische Wirkstoff, sofern er dem pastenförmigen Hämostyptikum zugesetzt wird, vorzugsweise einen Anteil an 0,5 Gew.-% bis 15,0 Gew.-% der Gesamtmasse des Wirkstofffreisetzungssystem ausmacht. Demnach kann der Anteil des partikulären pharmazeutischen Wirkstoffs an der Gesamtmasse des erfindungsgemä-ßen Wirkstofffreisetzungssystem mindestens 0,5 Gew.-%, mindestens 1,0 Gew.-%, mindestens 1,5 Gew.-%, mindestens 2,0 Gew.-%, mindestens 2,5 Gew.-%, ausmachen. Zusätzlich kann der Anteil des partikulären pharmazeutischen Wirkstoffs an der Gesamtmasse des erfindungsgemäßen Wirkstofffreisetzungssystem höchstens 15 Gew.-%, höchstens 14 Gew.-%, höchstens 13 Gew.-%, höchstens 12 Gew.-%, höchstens 11 Gew.-% höchstens 10 Gew.-%, ausmachen.

Bevorzugt umfasst das erfindungsgemäße Wirkstofffreisetzungssystem 1 Gew.-% bis 12 Gew.-%, besonders bevorzugt 2 Gew.-% bis 10 Gew.-% des partikulären pharmazeutischen Wirkstoffs.

Das erfindungsgemäße partikuläre Hämostyptikum oder das erfindungsgemäße Wirkstofffreisetzungssystem mit den jeweils beschriebenen Bestandteilen ist vorzugsweise so ausgebildet, dass es in destilliertem Wasser bei Raumtemperatur über einen Zeitraum von mindestens 7 Tagen unter Laborbedingungen formstabil bleibt. Dadurch ist es geeignet, in einer Umgebung, in der es dem Einfluss von polaren Flüssigkeiten ausgesetzt ist, seine gewünschte Form beizubehalten. Dies ist beispielsweise der Fall, wenn das erfindungsgemäße partikuläre Hämostyptikum oder das erfindungsgemäße Wirkstofffreisetzungssystem auf einen verletzten Knochen aufgetragen wird, um eine gegeben falls auftretende Blutung zu stillen.

Ein erfindungsgemäßer Vorteil ist, dass dem beanspruchten partikulären Hämostyptikum oder Wirkstofffreisetzungssystem keine anorganischen Calciumsalze und Magnesiumsalze hinzugesetzt werden müssen. Im Stand der Technik bekannte Hämostyptika enthalten diese anorganischen Salze häufig und haben den Nachteil, dass sie typischerweise eine Mohshärte ≥ 2 aufweisen. Durch diese hohe Mohshärte wirken sie abrasiv auf das sie umgebende Material ein. Dadurch, dass dem erfindungsgemäßen partikulären Hämostyptikum oder dem erfindungsgemäßen Wirkstofffreisetzungssystem vorzugsweise keine anorganischen Salze wie Calciumsalze und Magnesiumsalze hinzugesetzt werden müssen, besitzt das erfindungsgemäße partikuläre Hämostyptikum oder das erfindungsgemäße Wirkstofffreisetzungssystem keine abrasiven Eigenschaften und kann daher auch im Bereich von Gelenkendoprothesen eingesetzt werden, ohne dass die Gleitflächen durch Abrasion beeinflusst werden.

In einer Ausführungsform der vorliegenden Erfindung weist das erfindungsgemäße Hämostyptikum und das erfindungsgemäße Wirkstofffreisetzungssystem keine anorganischen Calciumsalze und Magnesiumsalze auf. Im Sinne der vorliegenden Erfindung ist es jedoch in einer weiteren Ausführungsform dennoch möglich, dem erfindungsgemäßen partikulären Hämostyptikum oder dem erfindungsgemäßen Wirkstofffreisetzungssystem Calcium- oder Magnesiumsalze mit einer Mohshärte ≥ 2,0 hinzuzugeben. In diesem Fall übersteigt der Masseanteil an Calcium- oder Magnesiumsalzen mit einer Mohshärte ≥ 2,0, jeweils bezogen auf das erfindungsgemäße partikuläre Hämostyptikum oder das erfindungsgemäße Wirkstofffreisetzungssystem, bevorzugt nicht mehr als 10,0 Gew.-%. Das bedeutet, bevorzugt werden weniger als 10,0 Gew.-%, weiter bevorzugt weniger als 9,5 Gew.-%, weiter bevorzugt weniger als 9,0 Gew.-%, weiter bevorzugt weniger als 8,5 Gew.-%, weiter bevorzugt weniger als 8,0 Gew.-%, weiter bevorzugt weniger als 7,5 Gew.-%, weiter bevorzugt weniger als 7,0 Gew.-%, weiter bevorzugt weniger als 6,5 Gew.-%, weiter bevorzugt weniger als 6,0 Gew.-%, weiter bevorzugt weniger als 5,5 Gew.-%, weiter bevorzugt weniger als 5,0 Gew.-% Calcium- oder Magnesiumsalze mit einer Mohshärte ≥ 2,0, jeweils bezogen auf das Gesamtgewicht des erfindungsgemäßen Hämostyptikums oder erfindungsgemäßen Wirkstofffreisetzungssystems, zugesetzt.

Dem erfindungsgemäßen partikulären Hämostyptikum oder dem erfindungsgemäße Wirkstofffreisetzungssystem können gemäß den vorstehenden Ausführungen insbesondere solche Calcium- oder Magnesiumsalze hinzugesetzt werden, die eine Mohshärte kleiner als 2 aufweisen, also eine Mohshärte von insbesondere kleiner als 2,0, insbesondere kleiner als 1,9, insbesondere kleiner als 1,8, insbesondere kleiner als 1,7, insbesondere kleiner als 1,6, insbesondere kleiner als 1,5, insbesondere kleiner als 1,4, insbesondere kleiner als 1,3, insbesondere kleiner als 1,2, insbesondere kleiner als 1,1, insbesondere kleiner als 1,0, insbesondere kleiner als 0,9, insbesondere kleiner als 0,8, insbesondere kleiner als 0,7, insbesondere kleiner als 0,6, insbesondere kleiner als 0,5, insbesondere kleiner als 0,4, insbesondere kleiner als 0,3, insbesondere kleiner als 0,2, insbesondere kleiner als 0,1.

Zusammenfassend weist das erfindungsgemäße Hämostyptikum sowie das erfindungsgemäße Wirkstofffreisetzungssystem folgende Vorteile auf:
Das erfindungsgemäße pastenförmige Hämostyptikum umfasst biodegradierbare Stoffe, die gemeinsam eine verformbare Paste bilden können, welche unter Krafteinwirkung irreversibel in eine gewünschte Form gebracht werden kann. Die Paste ist zum Einsatz in vivo geeignet und wenn sie dort abgebaut wird, bildet sie üblicherweise keine aciden oder basischen Abbauprodukte, welche lokale Schädigungen auslösen könnten.

Dem pastenförmigen Hämostyptikum können auch alle Arten denkbarer, partikulärer pharmazeutischer Wirkstoffe, wie vorstehend beschrieben, zugesetzt werden. In dieser Variante eignet es sich das resultierende Wirkstofffreisetzungssystem besonders zur Behandlung von Schädigungen des Knochengewebes jeglicher Art.

Das pastenförmige Hämostyptikum oder Wirkstofffreisetzungssystem bleibt nach der Aufbringung im Allgemeinen über einen längeren Zeitraum formstabil, auch wenn es flüssigem Medium ausgesetzt ist. Weiterhin müssen ihm vorzugsweise keine anorganischen Partikel hinzugesetzt werden, sodass es keinerlei abrasive Eigenschaften, beispielsweise gegenüber metallenen Objekten, aufweist.

All diese Eigenschaften lassen die Verwendung des erfindungsgemäßen pastenförmigen Hämostyptikum und Wirkstofffreisetzungssystem zur Versorgung aller Arten von Schädigungen am Knochengewebe zu.

### iii) Verwendungen des pastenförmigen Hämostyptikums

Das pastenförmige Hämostyptikum sowie das Wirkstofffreisetzungssystem haften im Allgemeinen bei Druckbeaufschlagung an Knochengewebe und an Metalloberflächen an. Dadurch ist es beispielsweise möglich, dass pastenförmige Hämostyptikum zu verwenden, um geschädigtes Knochengewebe zu behandeln, z. B., indem eine Blutung eines Knochens wie nach einem Bruch, einer Operation oder gar einer Amputation mit dem pastenförmigen Hämostyptikum gestoppt wird.

Da es üblicherweise ebenfalls an Metalloberflächen haftet, ist das pastenförmige Hämostyptikum auch insbesondere zur mechanischen Hämostase und/oder als pastenförmiges lokales Wirkstofffreisetzungssystem, das an Knochengewebeoberflächen und an Oberflächen von metallischen Implantaten durch Druckbeaufschlagung angeklebt wird, geeignet. Das pastenförmige lokale Wirkstofffreisetzungssystem ist, aufgetragen auf eine behandelte Stelle an einem Knochen, im Allgemeinen in der Lage, den dispergierten partikulären pharmazeutischen Wirkstoff freizugeben. Dabei ist das lokale Wirkstofffreisetzungssystem bevorzugt an die Oberflächen von Kniegelenkendoprothesen, Hüftgelenkendoprothesen, Schultergelenkendoprothesen, Marknägeln und Osteosyntheseplatten, vorzugsweise unter Druckeinwirkung, angeklebt. Ein großer Vorteil des erfindungsgemäßen Hämostyptikums ist dabei, dass es als lokales Wirkstofffreisetzungssystem nach dem Ankleben in-vivo üblicherweise ortsfest bleibt. Dadurch können im lokalen Wirkstofffreisetzungssystem dispergierte feste, vorzugsweise partikuläre, pharmazeutische Wirkstoffe gezielt am gewünschten Ort freigesetzt werden.

Weiterhin ist die Verwendung des pastenförmigen Hämostyptikums zur Auffüllung von Knochenkavitäten erfindungsgemäß möglich, wobei besonders bevorzugt das Hämostyptikum zur Auffüllung von zuvor infizierten und debridierten Markräumen und Schraubenbohrlöchern im Knochengewebe verwendet wird. Insbesondere dann, wenn das pastenförmige Hämostyptikum als lokales Wirkstoffzentrum auf solche Stellen aufgetragen wird, kann es den partikulären pharmazeutischen Wirkstoff abgeben und dann beispielsweise Entzündungssymptome lindern.

Das erfindungsgemäße pastenförmige Hämostyptikum wird insbesondere dazu verwendet, Blutungen des Knochens in-vivo mechanisch zu stoppen. Wenn dem erfindungsgemäßen pastenförmigen Hämostyptikum ein partikulärer pharmazeutischer Wirkstoff hinzugesetzt wird, kann es außerdem als pastenförmiges lokales Wirkstofffreisetzungssystem an der mit ihm behandelten Stelle dienen. Dort kann es dann über einen gewissen Zeitraum lang kontinuierlich den in ihm dispergierten pharmazeutischen Wirkstoff abgeben.

Daher betrifft die vorliegende Erfindung des Weiteren ein wie zuvor beschriebenes pastenförmiges Hämostyptikum oder ein wie zuvor beschriebenes pastenförmiges Wirkstofffreisetzungssystem zur Verwendung als Medikament, insbesondere zur Verwendung bei der Behandlung von beschädigtem Knochengewebe.

Das pastenförmige Hämostyptikum oder das pastenförmige Wirkstofffreisetzungssystem kann zur Verwendung bei der Behandlung von Knochenkavitäten durch Auffüllung, bevorzugt durch Auffüllung von infizierten und debridierten Markräumen und Schraubenbohrlöchern im Knochengewebe, verwendet werden.

Hierzu kann das pastenförmige Hämostyptikum oder pastenförmige Wirkstofffreisetzungssystem an Knochengewebeoberflächen und an Oberflächen von metallischen Implantaten durch Druckbeaufschlagung angeklebt wird, wobei das pastenförmige Hämostyptikum oder pastenförmige Wirkstofffreisetzungssystem bevorzugt an die Oberflächen von Kniegelenkendoprothesen, Hüftgelenkeendoprothesen, Schultergelenkendoprothesen, Marknägeln und Osteosyntheseplatten angeklebt wird.

Das pastenförmige Hämostyptikum kann manuell oder mithilfe einer Austragsvorrichtung an einen Patienten verabreicht werden. Aufgrund der enthaltenen Trilgyceride ist eine gute Gleitfähigkeit und Auspressbarkeit des Hämostyptikums erzielbar, beispielsweise in Spritzen- und ähnlichen Auspress-Dosiersystemen.

Die hierin beschriebenen Zusammensetzungen können auch zur Beschichtung medizinischer Implantate, beispielsweise Spacer, künstliche Gelenke und Osteosyntheseplatten, verwendet werden. Hierzu können die erfindungsgemäßen Zusammensetzungen beispielsweise als Applikationsstift oder Spritzensystem bereitgestellt werden.

### iv) Verfahren zur Herstellung des pastenförmigen Hämostyptikums und des Wirkstofffreisetzungssystem

Im Sinne der Erfindung wird das pastenförmige Hämostyptikum sowie Wirkstofffreisetzungssystem mittels Vermengung aller Bestandteile hergestellt.

Das bedeutet, dass mindestens ein partikulärer Zuckeralkohol a), mindestens ein gesättigter Glycerintrifettsäureester mit einem Schmelzpunkt größer/gleich 40 °C b), mindestens ein gesättigter Glycerintrifettsäureester mit einem Schmelzpunkt unter 0 °C c) sowie gegebenenfalls ein partikulärer pharmazeutischer Wirkstoff in einen geeigneten Behälter, wie ein Becherglas oder auch einen industriellen Mischbehälter, gegeben und vermengt werden.

Bevorzugt werden dabei 30 Gew.-% bis 60 Gew.-% des partikulären Zuckeralkohols, 20 Gew.-% bis 35 Gew.-% des gesättigten Glycerintrifettsäureesters mit einem Schmelzpunkt größer/gleich 40 °C, 20 Gew.-% bis 35 Gew.-% des gesättigten Glycerintrifettsäureesters mit einem Schmelzpunkt unter 0 °C und gegebenenfalls 0,5 bis 15 Gew.-% eines partikulären pharmazeutischen Wirkstoff eingewogen.

Bevorzugte Mengenverhältnisse der einzelnen Bestandteile sind bereits vorstehend offenbart und gelten für das erfindungsgemäße Verfahren *mutatis mutandis.*

Es kann im Sinne der Erfindung von Vorteil sein, wenn die eingewogenen Komponenten a), b), c) und gegebenenfalls d) zunächst erwärmt und unter geringerer Drehzahl vorgerührt werden. Die Erwärmung wird dabei im Allgemeinen über der Schmelztemperatur des gesättigten Glycerintrifettsäureesters mit einem Schmelzpunkt grö-βer/gleich 40 °C ausgeführt. Vorzugsweise ist sie nicht so hoch, dass die chemische Gestalt der angewendeten Bestandteile sich möglicherweise verändern könnte. Beispielsweise liegt sie zwischen 50 °C und 90 °C, also insbesondere bei 50 °C, 51 °C, 52 °C, 53 °C, 54 °C, 55 °C, 56 °C, 57 °C, 58 °C, 59 °C oder aber 60 °C, 61 °C, 62 °C, 63 °C, 64 °C, 65 °C, 66 °C, 67 °C, 68 °C, 69 °C oder aber 70 °C, 71 °C, 72 °C, 73 °C, 74 °C, 75 °C, 76 °C, 77 °C, 78 °C, 79 °C oder aber 80 °C, 81 °C, 82 °C, 83 °C, 84 °C, 85 °C, 86 °C, 87 °C, 88 °C, 89 °C oder aber 90 °C.

Im Anschluss wird das vorgerührte Gemisch in der Regel abgekühlt und mittels eines dazu geeigneten Misch- oder Rührgerätes bei hoher Drehzahl mindestens einmal gemischt. Dabei sind Drehzahlen zwischen 1500 min⁻¹ und 3000 min⁻¹ denkbar, beispielsweise 1500 min⁻¹, 1600 min⁻¹, 1700 min⁻¹, 1800 min⁻¹, 1900 min⁻¹, 2000 min⁻¹, 2100 min⁻¹, 2200 min⁻¹, 2300 min⁻¹, 2400 min⁻¹, 2500 min⁻¹, 2600 min⁻¹, 2700 min⁻¹, 2800 min⁻¹, 2900 min⁻¹ oder 3000 min⁻¹. Geeignet ist prinzipiell jedes Rührgerät, mit welchem hohe Rührzahlen, wie dargestellt, erreicht werden können. Im Sinne der vorliegenden Erfindung kann der Ruhrschritt bei erhöhter Drehzahl einmal durchgeführt oder mehrfach wiederholt werden.

Es ist im Sinne der vorliegenden Erfindung möglich, dem pastenförmigen Hämostyptikum einen partikulären pharmazeutischen Wirkstoff hinzuzusetzen, um es später als pastenförmiges lokales Wirkstofffreisetzungssystem zu verwenden. In so einem Fall kann der partikuläre pharmazeutische Wirkstoff zu Beginn gemeinsam mit den anderen Bestandteilen a), b) und c) eingewogen werden.

Das erfindungsgemäße Verfahren zur Herstellung des pastenförmigen Hämostyptikums, welches als pastenförmiges lokales Wirkstofffreisetzungssystem eingesetzt werden soll, ist insbesondere dadurch charakterisiert, dass mindestens ein im festen Aggregatzustand vorliegender, vorzugsweise partikulärer, pharmazeutische Wirkstoff durch Verkneten mit dem pastenförmigen Hämostyptikum vermischt wird.

### BESCHREIBUNG DER FIGUREN

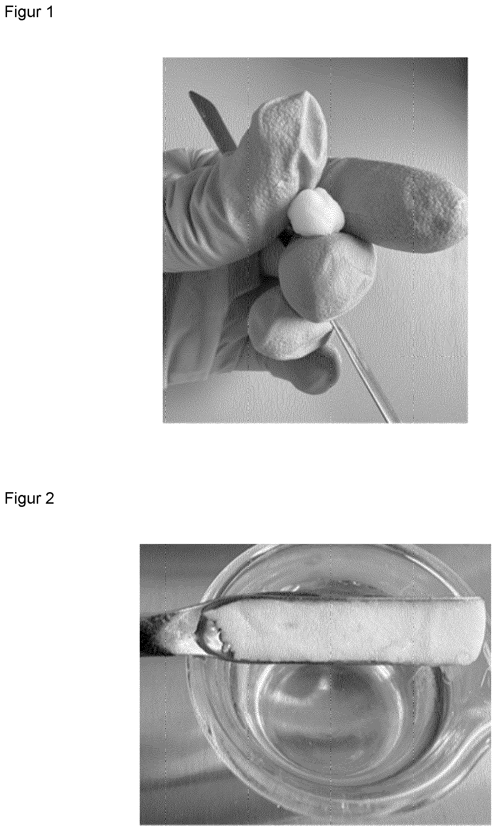

- **Figur 1**: Pastenförmiges Hämostyptikum fertiggestellt nach dem Vermischen aller Bestandteile, die Verformbarkeit in der Hand ist angedeutet.
- **Figur 2**: Pastenförmiges Hämostyptikum welches an die metallene Oberfläche eines Spatels angedrückt worden ist. Es ist ersichtlich, dass das pastenförmige Hämostyptikum an einer Metallfläche haften bleibt.

### AUSFÜHRUNGSBEISPIELE

Für die Ausführungsbeispiele wurden Lytrol micro, Poloxamer 185, Glycerintripalmitat, Glycerintristearat, Glycerintribehnat, Miglyol 812N und Miglyol 810N in Pharmaqualität verwendet.

Die Herstellung der Pasten erfolgte in einfacher Weise, dass zuerst alle Komponenten in ein Becherglas eingewogen wurden. Die Gemische wurden dann für 1 Stunde auf 90 °C erwärmt, wobei die Gemische gelegentlich gerührt wurden. Nach Abkühlung wurden die Gemische in Plastikdosen überführt und mit einem Speedmixer bei 2000 Umdrehungen für jeweils 60 Sekunden gemischt. Der Mischprozess wurde anschließend zweimal wiederholt. Es entstanden farblose pastöse Massen.

**Tabelle 1: Erfindungsgemäße Wirkstofffreisetzungssysteme unter Verwendung von D-Mannitol und Glycerintripalmitat**

| Beispiele | D-Mannitol [g] | Glycerintripalmitat [g] | Miglyol 812N [g} | Beurteilung |
|---|---|---|---|---|
| 1 | 9,1 | 4,6 | 4,4 | Sehr weich, haftet sehr gut auf Metall, Glas und Knochen |
| 2 | 9,1 | 4,6 | 4,0 | Weich, etwas fester als Paste des Beispiels 1. Haftet sehr gut auf Metall, Glas und Knochen |
| 3 | 9,1 | 4,1 | 4,0 | Weich, etwas fester als die Pasten der Beispiele 1 und 2, haftet ähnlich gut wie die Pasten der Beispiele 1 und 2 |

**Tabelle 2: Erfindungsgemäße Wirkstofffreisetzungssysteme unter Verwendung von D-Mannitol und Glycerintristearat sowie Glycerintribehenat:**

| Beispiele | D-Mannitol [g] | Glycerintristearat [g] | Glycerintribehenat [g] | Miglyol 812N [g] | Miglyol 812 [g] |
|---|---|---|---|---|---|
| 4 | 9,1 | 4,6 | - | 4,4 | |
| 5 | 9,1 | - | 4,6 | 4,0 | |
| 6 | 9,1 | 4,6 | - | 4,4 | |
| 7 | 9,1 | - | 4,6 | 4,0 | |

**Tabelle 3: Erfindungsgemäße Wirkstofffreisetzungssysteme unter Verwendung von Xylitol und Erythrit und Glycerintripalmitat**

| Beispiele | Xylitol [g] | Erythrit [g] | Glycerintripalmitat | Glycerintristearat [g] | Miglyol 812N |
|---|---|---|---|---|---|
| 8 | 9,1 | - | 4,6 | | 4,4 |
| 9 | 9,1 | - | 4,6 | | 4,4 |
| 10 | - | 9,1 | - | 4, | 4,4 |
| 11 | - | 9,1 | - | 4,6 | 4,4 |

**Tabelle 4: Referenzbeispiele gemäß der Lehre der Patentschrift DE 10 2011 016 277 B**

| Beispiele | Lytrol micro (Poloxamer) [g] | CSCA-Gemisch [g] | Glycerintripalmitat [g] | Miglyol 812N [g} | Beurteilung |
|---|---|---|---|---|---|
| 12 | 11,3 | | 5,6 | 9,0 | Sehr weich, deutlich weicher als die Paste des Beispiels 9, haftet sehr gut auf Metall, Glas und Knochengewebe |

| | | | | | |
|---|---|---|---|---|---|
| Das CSCA-Gemisch besteht aus 80,0 Gew.-% Calciumsulfat-Dihydrat und 20,0 Gew.-% Calciumcarbonat. | | | | | |

### Prüfung der in vitro-Cytotoxizität gemäß ISO10993-5

Es wurden Pasten der Beispiele 1, 2, und 12 hinsichtlich ihrer *in* vitro-Cytotoxizität untersucht. Weiterhin wurde das Poloxamer 185 mit in die Untersuchungen einbezogen.

Die Bestimmung der *in vitro*-Cytotoxizität gemäß der ISO10993-5 erfolgte mittels des MTT-Tests durch das Institut Eurofins BioPharma Product Testing Munich GmbH. Die Pasten wurden 72 Stunden bei 37 °C in Zellkulturmedium eluiert. Die Eluate wurden dann unverdünnt und in drei Verdünnungsstufen mit L929-Zellen 24 Stunden bei 37 °C inkubiert. Die Bestimmung der Vitalität der Zellen erfolgte dann unter Verwendung von Tetrazoliumchlorid photometrisch. Das Tetrazoliumchlorid wird dabei von vitalen Zellen zum rot-violetten Farbstoff 1,3,5-Triphenylformazan reduziert. Bei der Paste des Beispiels 12 wurde anstelle des Tetrazoliumchlorids der ähnlich aufgebaute Farbstoff Triphenyltetrazliumchlorid (XTT-Test) eingesetzt, der ebenfalls durch vitale Zellen zu einem rot-violetten Farbstoff reduziert wird. Dieser Farbstoff wird auch in der ISO10993-5 für die Prüfung der *in* vitro-Cytotoxizität beschrieben.

**Tabelle 5: Ergebnisse in vitro-Cytotoxizität für ausgesuchte Beispiele**

| Beispiele | Viability [%] | | | |
|---|---|---|---|---|
| | Test Extrakt-Verdünnung | | | |
| | 100% v/v | 69,6 %v/v | 44,4 % v/v | 29,6 % v/v |
| 1 | 73 | 74 | 79 | 82 |
| 2 | 72 | 79 | 81 | 82 |
| 12 | 44 | 49 | 65 | 77 |
| Poloxamer 185 | 0 | 0 | 0 | 1 |

Als nicht cytotoxisch werden gemäß ISO10993-5 die Proben bewertet, die bei 100% v/v eine Viability von größer 70 % beim MTT-Test oder beim XTT-Test besitzen. Die erfindungsgemäßen Pasten der Beispiele 1 und 2 waren gemäß dem MTT-Test mit 73 % und 72 % Viability als nicht cytotoxisch zu bewerten. Die Paste des Beispiels 12, die Poloxamer 185 enthält, war mit einer Viability kleiner 70 % eindeutig cytotoxisch.

Die *in* vitro-Cytotoxizität der Pasten der Beispiele 1 und 2 wurde dann zusätzlich mit dem Agar-Diffusionstest gemäß der ISO109992-5 unter Verwendung von L929-Zellen geprüft. Beide Pasten zeigten keinen cytotoxischen Effekt.

### Beurteilung der Zumischbarkeit von Antibiotika

Im Folgenden wurde die Zumischbarkeit von Antibiotika zum erfindungsgemäßen Pastenmaterial des Beispiels 1 geprüft. Dabei wurden die pulverförmigen Antibiotika Gentamicinhydrochlorid, Vancomycinhydrochlorid, Clindamycinhydrochlorid und Daptomycin verwendet. Es wurden jeweils 5,0 g Paste des Beispiel 1 mit 0,5 g des pulverförmigen Antibiotikums verknetet. Die haptischen Eigenschaften und die Haftung auf einer Stahloberfläche (1.4404 Stahl) wurden geprüft. Es zeigte sich, dass die eingekneteten Antibiotika nur einen geringen Effekt auf die Weichheit und Knetbarkeit hatten im Vergleich zur reinen Paste des Beispiels 1. Die Knetbarkeit ähnelte der Paste des Beispiels 2. Alle Pasten hafteten sehr gut auf 1.4404-Stahl. Die Haftung war ähnlich der Paste des Beispiels 2.

**Tabelle 6: Ergebnisse zur Zumischbarkeit von Antibiotika**

| | | Zusammensetzung | | Haptische Beurteilung | Haftung auf Stahl |
|---|---|---|---|---|---|
| Beispiel | | Paste | Antibiotikum | | |
| 13 | | 5,0 g Paste Beispiel 1 | 0,5 g Gentamicinsulfat | Weich, jedoch etwas weniger als reine Paste des Beispils1, leicht knetbar | Haftet sehr gut, ähnlich der reinen Paste des Beispiels 1 |
| 14 | | 5,0 g Paste Beispiel 1 | 0,5 g Vancomycinhydrochlorid | Weich, leicht knetbar, ähnlich der Paste des Beispiels 2 | Haftet sehr gut, ähnlich der reinen Paste des Beispiels 1 |
| 15 | | 5,0 g Paste Beispiel 1 | 0,5 g Clindamycinhydrochlorid | Weich, leicht knetbar, ähnlich der Paste des Beispiels 2 | Haftet sehr gut, ähnlich der reinen Paste des Beispiels 1 |
| 16 | | 5,0 g Paste Beispiel 1 | 0,5 g Daptomycin | Weich, leicht knetbar, ähnlich der Paste des Beispiels 2, aber geringfügig fester | Haftet sehr gut, ähnlich der reinen Paste des Beispiels 1 |

### Prüfung der Anhaftung an Metalloberflächen in Gegenwart von Wasser

Es wurde die Pasten an Metalloberflächen (1.4404 Stahl) geprüft. Dazu wurde ca. 2 g Proben der Beispiele auf eine Spateloberfläche gedrückt. Die Pasten hafteten an der Metalloberfläche. Die am Metall anhaftenden Pasten wurde 7 Tage bei Raumtemperatur in Wasser gelagert. Es erfolgte keine Ablösung. Der pH-Wert des destillierten Wassers lag im Bereich von pH 6,0-6,8.

## Patentansprüche

1. Pastenförmiges Hämostyptikum, umfassend
a) mindestens einen partikulären Zuckeralkohol;
b) mindestens einen gesättigten Glycerintrifettsäureester mit einem Schmelzpunkt größer/gleich 40 °C; und
c) mindestens einen gesättigten Glycerintrifettsäureesters mit einem Schmelzpunkt von kleiner 0 °C.

2. Pastenförmiges Hämostyptikum nach Anspruch 1, umfassend, jeweils bezogen auf das Gesamtgewicht des Hämostyptikums,
a) 30 bis 60 Gew.-% mindestens eines partikulären Zuckeralkohols;
b) 20 bis 35 Gew.-% mindestens eines gesättigten Glycerintrifettsäureesters mit einem Schmelzpunkt größer/gleich 40 °C; und
c) 20 bis 35 Gew.-% mindestens eines gesättigten Glycerintrifettsäureesters mit einem Schmelzpunkt von kleiner 0 °C.

3. Pastenförmiges Hämostyptikum nach Anspruch 1 oder 2, **dadurch gekennzeichnet, dass** es eine Viability von größer 70 % im MTT- oder XTT- in-vitro Cytotoxizitätstest gemäß der ISO10993-5 Norm bei 100% v/v aufweist.

4. Pastenförmiges Hämostyptikum nach einem der Ansprüche 1 bis 3, **dadurch gekennzeichnet, dass** der partikuläre Zuckeralkohol aus Alditolen und Ketolen ausgewählt ist.

5. Pastenförmiges Hämostyptikum nach einem der Ansprüche 1 bis 4, **dadurch gekennzeichnet, dass** der gesättigte Glycerintrifettsäureester mit einem Schmelzpunkt größer/gleich 40 °C ausgewählt ist aus Glycerintristearat, Glycerintripalmitat, Glycerintrilaurat und gemischten Glycerintrifettsäureester, die aus Stearat und/oder Palmitat und/oder Laurat und Glycerin gebildet sind.

6. Pastenförmiges Hämostyptikum nach einem der Ansprüche 1 bis 5, **dadurch gekennzeichnet, dass** der gesättigte Glycerintrifettsäureester mit einem Schmelzpunkt von kleiner 0 °C ausgewählt ist aus Glycerintrioctanoat, Glycerintridecanoat oder den Glycerinfettsäuregemischen Miglyol 812 N, Miglyol 810 N und Miglyol 829 N.

7. Pastenförmiges Hämostyptikum nach einem der Ansprüche 1 bis 6, **dadurch gekennzeichnet, dass** der partikuläre Zuckeralkohol einen Partikeldurchmesser kleiner 100 µm aufweist, wobei die Partikeldurchmesser durch Siebfraktionierung bestimmt werden.

8. Pastenförmiges Hämostyptikum nach einem Ansprüche 1 bis 7, **dadurch gekennzeichnet, dass** das Hämostyptikum in destilliertem Wasser bei Raumtemperatur über einen Zeitraum von 7 Tagen formstabil bleibt.

9. Pastenförmiges Wirkstofffreisetzungssystem, umfassend ein pastenförmiges Hämostyptikum gemäß einem der Ansprüche 1 bis 8 sowie mindestens einen pharmazeutischen Wirkstoff, vorzugsweise einen partikulären pharmazeutischen Wirkstoff, wobei der pharmazeutische Wirkstoff vorzugsweise ausgewählt ist aus Antiinfektiva, Aktivatoren der Blutgerinnung, Fibrinolysehemmer, Immunmodulatoren, Steroidhormone und Wachstumsfaktoren.

10. Pastenförmiges Hämostyptikum gemäß einem der Ansprüche 1 bis 8 oder pastenförmiges Wirkstofffreisetzungssystem gemäß Anspruch 9 zur Verwendung als Medikament.

11. Pastenförmiges Hämostyptikum gemäß einem der Ansprüche 1 bis 8 oder pastenförmiges Wirkstofffreisetzungssystem gemäß Anspruch 9 zur Verwendung bei der Behandlung von beschädigtem Knochengewebe.

12. Pastenförmiges Hämostyptikum oder pastenförmiges Wirkstofffreisetzungssystem gemäß Anspruch 10 oder 11 zur Verwendung bei der Behandlung von Knochenkavitäten durch Auffüllung, bevorzugt durch Auffüllung von infizierten und debridierten Markräumen und Schraubenbohrlöchern im Knochengewebe.

13. Pastenförmiges Hämostyptikum oder pastenförmiges Wirkstofffreisetzungssystem gemäß einem der Ansprüche 10 bis 12, **dadurch gekennzeichnet, dass** das pastenförmige Hämostyptikum oder pastenförmige Wirkstofffreisetzungssystem an Knochengewebeoberflächen und an Oberflächen von metallischen Implantaten durch Druckbeaufschlagung angeklebt wird, wobei das pastenförmige Hämostyptikum oder pastenförmige Wirkstofffreisetzungssystem bevorzugt an die Oberflächen von Kniegelenkendoprothesen, Hüftgelenkeendoprothesen, Schultergelenkendoprothesen, Marknägeln und Osteosyntheseplatten angeklebt wird.

14. Verfahren zur Herstellung eines pastenförmigen Hämostyptikums gemäß einem der Ansprüche 1 bis 8, **dadurch gekennzeichnet, dass** mindestens ein partikulärer Zuckeralkohol, mindestens ein gesättigter Glycerintrifettsäureester mit einem Schmelzpunkt größer/gleich 40 °C und mindestens ein gesättigter Glycerintrifettsäureester mit einem Schmelzpunkt von kleiner 0 °C in einen Mischbehälter gegeben und miteinander verknetet werden.

15. Verfahren nach Anspruch 14, **dadurch gekennzeichnet, dass** mindestens ein partikulärer pharmazeutischer Wirkstoff zusätzlich durch Verkneten mit dem pastenförmigen Hämostyptikum vermischt wird.
